# EUROPÄISCHE PATENTSCHRIFT

(11) **EP 0 321 884 B1**
(45) Veröffentlichungstag und Bekanntmachung des Hinweises auf die Patenterteilung: **24.11.1993**
(21) Anmeldenummer: 88121140.3
(22) Anmeldetag: 16.12.1988
(51) Int. Cl.: A61B 17/28, A61B 17/58

(54) **Zange**
Forceps
Pince

(30) Priorität: 22.12.1987 DE 3743605
(43) Veröffentlichungstag der Anmeldung: 28.06.1989
(73) Patentinhaber: DELMA ELEKTRO-UND MEDIZINISCHE APPARATEBAU GESELLSCHAFT mbH, D-78532 Tuttlingen (DE)
(72) Erfinder: Mäntele, Erwin, D-7200 Tuttlingen (DE)
(74) Vertreter: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) Entgegenhaltungen:
- AT-B- 367 631
- DE-A- 3 119 550
- DE-B- 2 244 333
- DE-U- 7 529 050
- US-A- 4 462 403

## Beschreibung

Die Erfindung betrifft eine Zange, insbesondere für chirurgische Zwecke nach dem Oberbegriff des Patentanspurchs 1.

Bei knochen-chirurgischen Eingriffen besteht sehr oft die Notwendigkeit, Stahldrähte mit hoher Kerbschlagzähigkeit zu kürzen. So werden z.B. Kirschnerdrähte mit Durchmessern von 1,0 bis 3,0 mm zur Drahtextension so durch Gelenke gebohrt, daß sie beidseitig überstehen. Die gleichen Drähte werden als Führungsspieße zur Führung beim Eindrehen von Hohlschrauben benutzt. Außerdem werden z.B. in der Handchirurgie ganze Fingerflieder in Längsrichtung mit Kirschnerdrähten zu sicheren Fixierungen durchbohrt. Diese Kirschnerdrähte haben handelsübliche Längen und werden beim chirurgischen Eingriff an die Gegebenheiten angepaßt und gekürzt.

Ferner ist es üblich, bei Verwendung eines sogenannten Fixateurs externe Steinmanägel durch die Knochen von Extremitäten zu treiben. Diese Steinmannägel müssen dann für die Montage am Fixateur extern ebenfalls auf die richtige Länge gekürzt werden. Das gleiche gilt für Schanz'sche Schrauben.

In all diesen Fällen werden Zangen der eingangs genannten Art zur Materialtrennung benutzt, da sich das Absägen oder Durchschleifen der genannten Drähte bzw. Schrauben aus hygienischen Gründen verbietet. Die Materialtrennung muß somit spanlos erfolgen, da wegen des Infektionsrisikos Sägespäne oder Schleifstaub nicht in offene Wunden gelangen darf.

Die in der Chirurgie bisher zur Anwendung kommenden Zangen, die meistens die Form eines Seitenschneiders, seltener die eines Bolzenschneiders mit stirnseitigen Schneiden haben, besitzen zwischen den Griffhebeln ein Getriebe, um den Operateur die notwendige Kraftausübung auf die Schneiden der Stange zu ermöglichen.

Da durch die Anatomie der menschlichen Hand und dem Durchmesser der durchzutrennenden Drähte bzw. Schrauben der Öffnungswinkel der Zangengriffe begrenzt ist, werden der Kraftübersetzung durch das Getriebe natürliche Grenzen gesetzt. Hierdurch ist der Operateur sehr oft gezwungen, insbesondere zum Trennen von Drähten und Schrauben mit größerem Durchmesser beide Hände einzusetzen, um so die nötige Trennkraft zu erzeugen. Dies ist jedoch relativ umständlich und aufwendig.

Der Erfindung liegt nun die Aufgabe zugrunde, eine chirurgische Zange der eingangs genannten Art zu schaffen, die es ermöglicht, selbst Drähte und Schrauben mit größerem Durchmesser bereits bei einhändiger Bedienung einfach und mit vertretbarem Kraftaufwand zu durchtrennen.

Diese Erfindung wird erfindungsgemäß durch Merkmale des kennzeichnenden Teils des Patentanspruchs 1 gelöst.

Durch diese erfindungsgemäß vorgesehenen Maßnahmen wird erreicht, daß bei gleicher auf die Handgriffe der Zange wirkender Kraft sich die von den Schneiden der Zange auf den Draht ausgeübte Trennkraft während des fortschreitenden Trennvorgangs vergrößert, so daß der Operateur nicht gezwungen ist beim Durchtrennen von Drähten oder Schrauben während der Operation die zweite Hand zum Durchtrennen zu benutzen. Hierdurch wird die Bedienung der Zange wesentlich vereinfacht und der Operateur hat die andere Hand für weitere Tätigkeiten frei.

Der Operateur kann die Zange zunächst bis zum Einklemmen des Werkstückes bei geringer Kraftübersetzung bewegen. Dabei legen die Schneiden relativ zueinander in bezug auf einen bestimmten Schließweg der Handgriffe einen relativ großen Weg zurück. Am Anfang des Schneidvorganges, wenn die an den Schneiden erforderliche Kraft hoch gering ist, ändert sich die Kraftübersetzung wenig, so daß die Scheiden immer noch einen relativ großen Weg zurücklegen. Erst während des Durchtrennens nimmt die Kraftübersetzung und damit auch die Kraft an den Schneiden erfindungsgemäß stark zu, während der von den Schneiden zurückgelegte Schließweg in bezug auf den Schließweg der Handgriffe stark abnimmt.

Dabei ist es von Vorteil, daß die Schneiden leicht geöffnet werden können, um ein Werkstück zu erfassen, ohne daß die Handgriffe übermäßig weit voneinander weggeschwenkt zu werden brauchen, da bei geöffneter Zange die Wegübersetzung noch relativ groß ist.

Durch die erfindungsgemäßen Maßnahmen kann erreicht werden, daß die Kraftübersetzung mit kleiner werdendem Abstand Zwischen dem Griffhebel und dem Schneiden-Griffhebel im wesentlichen quadratisch zunimmt. Hierdurch wird erreicht, daß - wiederum bei gleichbleibender auf die Zangengriffe wirkender Kraft - sich die von den Schneiden auf den zu durchtrennenden, insbesondere runden Gegenstand ausgeübte Kraft so erhöht, daß der zur Materialtrennung erforderliche Trenndruck der Schneiden auf den Gegenstand während des gesamten Trennvorgangs im wesentlichen gleich bleibt. Hierzu ist die erfindungsgemäß vorgesehene Erhöhung der Kraftübersetzung erforderlich, da die Auflagefläche der Schneiden beim Eindringen in den Gegenstand sich ebenfalls im wesentlichen quadratisch vergrößert. Die Erfindung ermöglicht somit neben dem sicheren Durchtrennen der Drähte oder Schrauben eine sehr gleichmäßige Kraftanwendung, so daß der Chirurg während der Operation die erfindungsgemäße Zange äußerst gezielt und gleichmäßig bedienen kann.

Bei einem bevorzugtem Ausführungsbeispiel der Erfindung ist vorgesehen, daß als Getriebe ein Kniehebelantrieb vorgesehen ist, dessen Antriebsglied von einem mit seinem ersten Ende am Griffhebel angelenkten Übertragungshebel gebildet ist, der zusammen mit dem Griffhebel einen ersten Kniehebel gebildet der mit seinem anderen Ende ein Kniegelenk eines zweiten Kniehebels beaufschlagt, welcher aus einem am Schneiden-Griffhebel angelenkten Stützhebel und einem Zwischenhebel besteht, der sich vom Kniegelenk zum Schneidenhebel erstreckt, an dem er in einem Zwischengelenk angelenkt ist. Hierdurch wird nun eine leicht zu bedienende Zange geschaffen, die konstruktiv einfach aufgebaut und daher preiswert zu fertigen ist.

Bei einer vorteilhaften Weiterbildung der Erfindung ist vorgesehen, daß zwischen dem Griffhebel und dem Übertragungshebel ein bei Betätigung der Zange sich verkleinernder, spitzer Winkel und zwischen dem Stützhebel und dem Zwischenhebel ein bei Betätigung der Zange sich vergrößernder, stumpfer Winkel vorgesehen ist, wobei bei geschlossenen Schneiden der Übertragungshebel nahezu parallel zum Griffhebel und der zweite Kniehebel kurz vor seiner Strecklage liegt. Durch diese spezielle Wahl der Winkel der beiden Kniehebel wird erreicht, daß die Kraftübersetzung im Endbereich der Schließbewegung der Zange besonders groß wird, da relativ große Winkeländerungen der Kniehebel nur noch eine sehr kleine Verschiebung ihrer Anlenkpunkte bewirken.

Um bei der erfindungsgemäßen Zange das erforderliche Kraftübersetzungsverhältnis auf einfache Weise an die jeweiligen Gegebenheiten anpassen zu können, ist bei einem weiteren Ausführungsbeispiel der Erfindung vorgesehen, daß der am Griffhebel angelenkten Übertragungshebel etwa 3 bis 6-mal, vorzugsweise 4 bis 5-mal, insbesondere 4,5-mal länger ist, als der Stützhebel, wobei der Abstand zwischen dem den Griffhebel mit dem Schneiden-Griffhebel verbindenden ersten Gelenk und dem zweiten Gelenk, das das Kniegelenk des ersten Kniehebels bildet, etwa 1,5 bis 3-mal, vorzugsweise 2 bis 2,5-mal größer ist als der Abstand zwischen dem ersten Gelenk und dem Stützgelenk, das den Stützhebel am Schneiden-Griffhebel hält.

Um einen besonders kompakten und handlichen Aufbau der Zange zu erreichen, der für deren Einsatz während einer Operation wichtig ist, sieht ein anderes Ausführungsbeispiel der Erfindung vor, daß dei Länge des Zwischenhebels etwa 1/6 bis 2/3, vorzugsweise 1/4 bis 1/2, insbesondere 1/3 der Länge des Übertragungshebels beträgt.

Bei einem weiteren bevorzugten Ausführungsbeispiel der Erfindung ist vorgesehen, daß die Länge des Zwischenhebels verkleinerbar ist, wobei der Zwischenhebel als Feder ausgebildet ist. Hierdurch läßt sich erreichen, daß beim Schließen der Zange zum Durchtrennen von Drähten bzw. Schrauben mit großen Durchmessern nach einem geringfügigem Einschneiden bzw. Einklemmen des Drahtes bzw. der Schraube mittels der Schneiden der Schließwinkel zwischen den Schneiden zunächst unverändert bleibt, während sich der Schließwinkel der Zangengriffe, also der Abstand zwischen dem Schneiden-Griffhebel und dem Griffhebel, so weit verkleinert, bis sich das Übersetzungsverhältnis für die von den Schneiden ausgeübte Kraft soweit vergrößert hat, daß der von den Schneiden der Zange ausgeübte Druck dem Trenndruck entspricht, so daß der Trennvorgang an dem zwischen den Zangenschneiden eingeklemmten Draht bzw. der Schraube fortgesetzt werden kann. Daher braucht der Operateur auch beim Durchtrennen dicker Drähte oder Schrauben nur die gleiche Kraft aufzuwenden, wie sie zum Durchtrennen dünner Drähte erforderlich ist. Es ergibt sich daher eine besonders gleichmäßige von der Dicke der zu durchtrennenden Gegenstände unabhängige Betätigung der erfindungsgemäßen Zange.

Um auch Gegenstände mit besonders hoher Kerbschlagzähigkeit durchtrennen zu können, sieht ein weiteres Ausführungsbeispiel der Erfindung vor, daß der Zwischenhebel von einer U-förmigen Druckfeder gebildet ist, deren Federschlitz die Längenveränderung des Zwischenhebels begrenzt, wobei die maximale Längenverkleinerung des Zwischenhebels etwa 1/10 bis 1/3, vorzugsweise 1/7 bis 1/4, insbesondere 1/5 des Gesamtverschiebeweges des Kniegelenks beträgt. Hierdurch wird gewährleistet, daß sich die Schneiden der Zange vollständig aneinander anlegen, wenn die Griffe der Zange vollständig zusammengedrückt werden. Somit ist stets eine einwandfreie Trennung von Werkstücken während der Operation gewährleistet.

Bei einem anderen Ausführungsbeispiel der Erfindung ist vorgesehen, daß das zweite, das Kniegelenk des ersten Kniehebels bildende Gelenk und/oder das Stützgelenk in Richtung des Griffhebels bzw. des Schneiden-Griffhebels verstellbar an diesen angeordnet sind. Hierdurch wird eine weitere Möglichkeit geschaffen, um beim Durchtrennen von Drähten bzw. Schrauben mit großen Durchmessern bereits nach einem geringfügigem Einschneiden bzw. Einklemmen des Werkstücks den Schließwinkel der Zangengriffe soweit zu verkleinern, bis sich das Kraftübersetzungsverhältnis so weit vergrößert hat, daß von den Schneiden der nötige Trenndruck auf das Werkstück ausgeübt werden kann.

Eine besonders einfache, nur mit einer Kniehebelanordnung arbeitende Ausführungsform kennzeichnet sich gemäß der Erfindung dadurch, daß der Griffhebel jenseits des Griffhebelgelenks durch einen relativ kurzen Kniehebelarm verlängert ist, der über ein Kniegelenk eine zusammen mit dem Kniehebelarm einen Kniehebel bildende Kniehebellasche beaufschlagt, die mit dem von Kniegelenk abgewandten Ende an dem bezüglich des Schneidenhebelgelenks von der Schneide abgewandten Arm des Schneidenhebels gelenkig verbunden ist, wobei der Kniehebel beim Verringern des Abstandes zwischen Griffhebel und Handgriff von einer stark geknickten in eine mehr gestreckte Postion übergeht und die Kniehebellasche (20) im geöffneten Zustand der Zange eine deutlichen Winkel von vorzugsweise wenigstens 30 bis 40°, insbesondere 35°, mit dem besagten Hebelarm (2′) einschließt,welcher sich zweckmäßig bei aufeinander zu bewegenden Schneiden (15, 15′) immer mehr an 90° annähert und/oder bei geschlossenen oder annähernd geschlossenen Schneiden (15, 15′) bevorzugt 40-80°, zweckmäßig 60-70° und insbesondere etwa 60° beträgt.

Hierbei ist insbesondere vorgesehen, daß der Kniehebel im unbetätigten Zustand der Zange einen Knickwinkel von größenordnungsmäßig 90° und vorzugsweise etwas weniger als 90°, insbesondere etwa 70 bis 80° aufweist und sich im vollständig zusammengedrückten Zustand der Zange nahe seinem gestreckten Zustand befindet und bevorzugt einen Knickwinkel zwischne 140° und 170°, bevorzugt 150° bis 160° aufweist.

Weiter ist es zweckmäßig, wenn der Hebelarm zwischen dem Schneidenhebelgelenk und der Kniehebellasche um einen Faktor 1,5 bis 2 länger ist als der die Schneide tragende Hebelarm.

Die Erfindung wird im folgenden beispielsweise anhand der Zeichnungen näher erläutert; in dieser zeigt:
- Fig. 1: eine Draufsicht auf eine chrirugische Zange im geöffneten Zustand,
- Fig. 2: eine Draufsicht auf die geschlossene chirurgische Zange nach Figur 1, wobei ein Teil weggebrochen ist,
- Fig. 3: eine vergrößerte schematische Darstellung der Schneiden einer chirurgischen Zange beim Durchtrennen eines Drahtes,
- Fig. 4: einen Schnitt im wesentlichen nach Linie IV-IV in Figur 3,
- Fig. 5: eine Darstellung der Schneiden einer chirurgischen Zange entsprechend Figur 3, wobei die Schneiden weiter geschlossen sind,
- Fig. 6: einen Schnitt im wesentlichen nach Linie VI-VI in Fig. 5,
- Fig. 7: eine Darstellung des Kraft-Übersetzungsverhältnisses der Zange in Abhängigkeit von deren Schließweg,
- Fig. 8: eine Seitenansicht einer besonders einfach aufgebauten Ausführungsform einer erfindungsgemäßen chirurgischen Zange im geöffneten Zustand und
- Fig. 9: eine entsprechende Seitenansicht wie Fig. 8, wobei die Zange sich jedoch im zusammengedrückten Zustand befindet.

In den verschiedenen Figuren der Zeichnung sind einander entsprechende Teile mit gleichen Bezugszeichen bezeichnet.

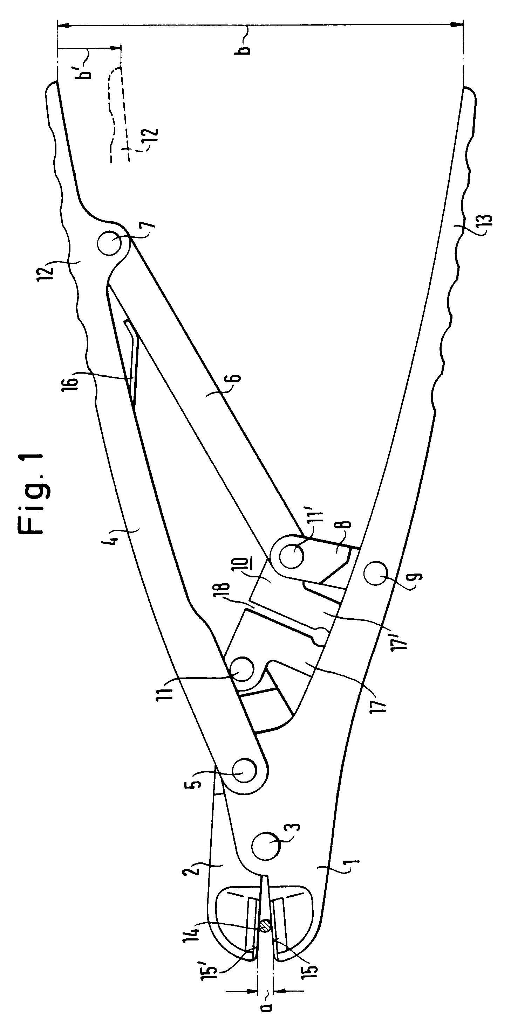

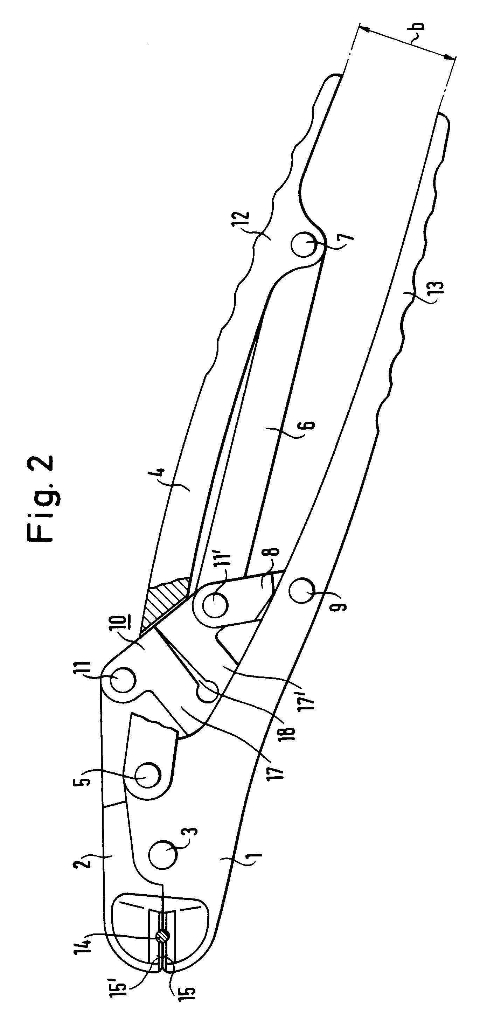

Entsprechend Figur 1 und 2 besitzt die chirurgische Zange einen Schneiden-Griffhebel 1, an dessen vorderem, in der Zeichnung linken Ende eine erste Schneide 15 und an dessen hinteren Ende ein Handgriff 13 vorgesehen ist. Ein Schneidenhebel 2 ist über ein Schneidenhebelgelenk 3 schwenkbar am Schneiden-Griffhebel 1 gelagert. An seinem vorderen Anschnitt weist der Schneidenhebel 2 eine zweite, mit der ersten Schneide 15 zusammenwirkende Schneide 15′ auf, wobei die beiden Schneiden 15, 15′ nach Art eines Seitenschneiders angeordnet sind.

Hinter dem Schneidenhebelgelenk 3, also auf der von den Schnieden 15, 15′ abgewandten Seite des Schneidenhebelgelenks 3 ist am Schneidengriffhebel 1 über ein Griffhebelgelenk 5 ein Griffhebel 4 angelenkt, der an seinem vom Griffhebelgelenk 5 abgewandten Ende einen Handgriff 12 aufweist. Im Bereich des Handgriffs 12 ist über ein weiteres Gelenk 7 ein Übertragungshebel 6 angebracht, der zusammen mit dem Griffhebel 4 einen ersten Kniehebel 4, 6 bildet und der an seinem anderen Ende auf ein Kniegelenk 11′ eines zweiten Kniehebels 8, 10 einwirkt, der von einem Stützhebel 8 und einem Zwischenhebel 10 gebildet ist.

Der Stützhebel 8 ist dabei schwenkbar über ein Stützgelenk 9 am Schneiden-Griffhebel 1 befestigt, das etwa in der Mitte zwischen dem Schneidenhebelgelenk 3 und dem Handgriff 13 des Schneiden-Griffhebels 1 angeordnet ist. Der am Kniegelenk 11′ angelenkte Zwischenhebel 10, der federnd zusammendrückbar ist, erstreckt sich vom Kniegelenk 11′ nach vorn und ist mit seinem vorderen Ende über ein Zwischengelenk 11 mit dem hinteren Ende des Schneidenhebels 2 verbunden.

Das Zwischenstück ist dabei im allgemeinen U-förmig ausgebildet, wobei der zwischen den Federschenkeln 17, 17′ des U's vorgesehene Federschlitz 18 im wesentlichen senkrecht zu der Verbindungslinie zwischen dem Zwischengelenk 11 und dem Kniegelenk 11′ verläuft.

Zwischen dem Griffhebel 4 und dem Übertragungshebel 6 weist der erste Kniehebel einen spitzen Winkel auf, der sich beim Zusammendrücken der Handgriffe 12, 13 verkleinert. Dabei wir der Übertragungshebel 6 mit seinem vorderen Ende nach vorne, also in Richtung der Schneiden 15, 15′ verschoben und bewirkt dadurch eine Verschiebung des Kniegelenks 11′, wobei ein zwischen dem Stützhebel 8 und dem Zwischenhebel 10 vorgesehener stumpfer Winkel des zweiten Kniehebels 8, 10 vergrößert wird.

Um die Zange selbsttätig in ihre geöffnete Stellung zu bringen, ist zwischen dem Griffhebel 4 und dem Übertragungshebel 6 eine Spreizfeder 16 vorgesehen, die den Übertragungshebel 6 vom Griffhebel 4 wegdrückt.

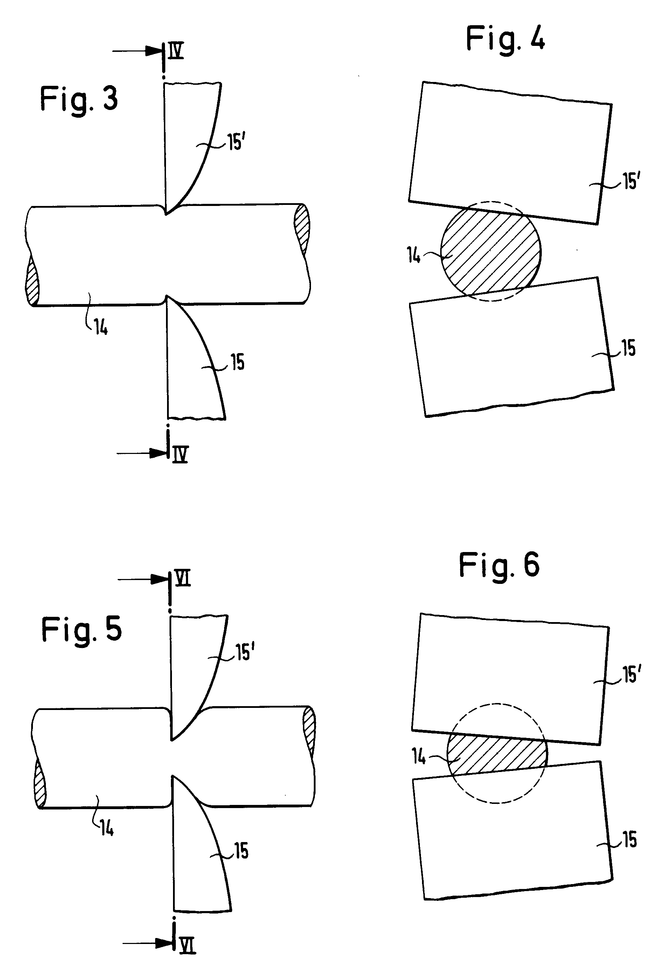

Im folgenden wird die Funktion der beschriebenen chirurgischen Zange unter Bezugnahme auf die Figuren 3 bis 6 im einzelnen näher erläutert:
Um einen Draht 14, eine Schraube oder ähnliches mit der beschriebenen chirurgischen Zange durchzutrennen, wird ein Draht 14 in üblicher Weise zwischen den Schneiden 15, 15′ angeordnet und anschließend werden die Handgriffe 12, 13 zusammengedrückt. Dabei wird das das Kniegelenk 11′ des zweiten Kniehebels 8, 10 nach vorne verschoben. Diese Bewegung wird vom federnden Zwischenhebel 10 über das Zwischengelenk 11 auf den Schneidenhebel 2 übertragen, der daraufhin in bezug auf den Schneiden-Griffhebel 1 im Gegenuhrzeigersinn um das Schneidenhebelgelenk 3 geschwenkt wird, so daß sich die beiden Schneiden 15, 15′ einander nähern und dabei in den zu trennenden Draht eindringen, wie dies in den Figuren 3 bis 6 dargestellt ist.

Dabei dringen in der Anfangsphase des Trennvorgangs die Schneiden 15, 15′ der Zange zunächst mit geringer Auflagefläche von beiden Seiten in den beispielhaft in der Zeichnung dargestellten runden Draht ein, wie die Figuren 3 und 4 zeigen. Wegen der zunächst geringen Auflagefläche der Schneiden 15, 15′ is in dieser Phase der Druck sehr hoch, so daß zunächst die Druckerzeugung keine Schwierigkeiten bereitet.

Mit dem weiteren Eindringen der Schneiden 15, 15′ in den Draht 14 vergrößert sich die Auflagefläche der Schneiden 15, 15′, so daß - solange die Schneiden 15, 15′ mit konstanter Kraft zusammengedrückt werden - der von den Schneiden 15, 15′ auf den Draht 14 ausgeübte Druck abnimmt. Hierdurch wird ab einer gewissen Eindringtiefe, die unter anderem vom Durchmesser des zu trennenden Drahtes 4 abhängt, der von den Schneiden 15, 15′ ausgeübte Druck so klein, daß er zum weiteren Trennen des Drahtes 14 nicht mehr ausreicht.

Um nun den Druck so groß zu halten, daß stets eine weitere Trennung des Drahtes 14 ermöglicht ist, muß nun die auf die Schneiden 15, 15′ wirkende Kraft entsprechend der Vergrößerung der Auflagefläche der Schneiden 15, 15′ ebenfalls vergrößert werden.

Die Figuren 5 und 6 zeigen nun die weiter in den Draht 14 eingedrungenen Schneiden 15, 15′ und insbesondere aus Figur 6 ist die Vergrößerung der Auflageflächen der Schneiden deutlich zu erkennen.

Es ergibt sich nun, daß die für eine vollständige Durchtrennung runder Körper, z. B. eines Drahtes 14, benötigte Kraft in Abhängigkeit von der Eindringtiefe der Schneiden 15, 15′ nahezu quadratisch anwachsen muß, um während des fortschreitenden Trennungsvorgangs stets den erforderlichen Trenndruck der Schneiden 15, 15′ zu erzeugen.

Bei der beschriebenen Zange verkleinert sich bei einem Trennvorgang der vom Griffhebel 4 und dem Schneiden-Griffhebel 1 gebildete Schließwinkel. Gleichzeitig verkleinert sich auch der zwischen dem Griffhebel 4 und dem Übertragungshebel 6 liegende Winkel des ersten Kniehebels 4, 6, bis bei geschlossener Zange der Übertragungshebel 6 nahezu parallel zum Griffhebel 4 liegt. Andererseits vergrößert sich der Winkel des zweiten Kniehebels 8, 10, so daß bei geschlossener Zange der zweite Kniehebel 8, 10 fast seine Strecklage einnimmt, und der Stützhebel 8 nahezu in einer Linie mit dem Zwischenhebel 10 liegt.

Dabei wird die Vorwärtsbewegung des Übertragungshebels 6 bzw. des Kniegelenks 11′ im Verhältnis zur Schließbewegung der Handgriffe 12, 13 immer geringer. Dementsprechend wird auch die Schwenkbewegung des Schneidenhebels 2 mit sich verkleinerndem Schließwinkel zwischen den Handgriffen 12, 13 immer geringer, so daß mit kleiner werdendem Abstand b zwischen den Handgriffen 12, 13 für gleiche Änderungen des Abstandes b immer kleinere Änderungen des Abstandes a zwischen den Schneiden 15, 15′ erhalten werden. Damit vergrößert sich das Übersetzungsverhältnis für die auf die Handgriffe 12, 13 wirkende Kraft, so daß die auf die Schneiden 15, 15′ wirkende Kraft vergrößert wird, ohne daß Vergrößerung der Kraft auf die Handgriffe 12, 13 erforderlich ist.

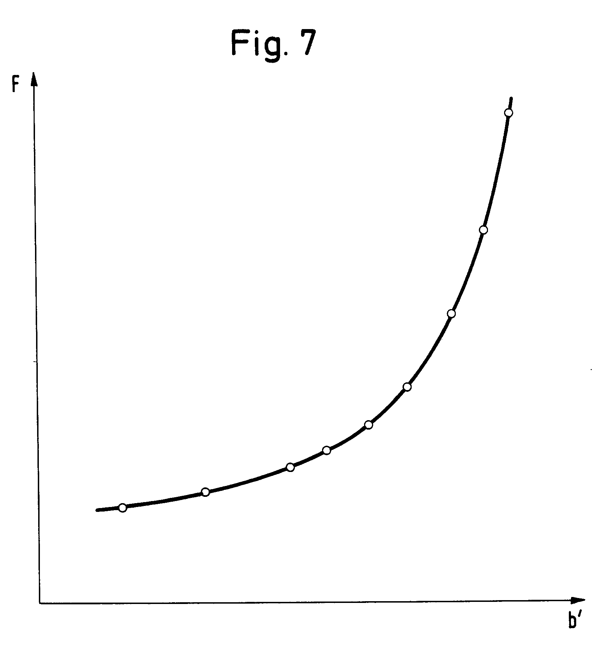

In Figur 7 ist nun die auf die Schneiden 15, 15′ ausgeübte Kraft F für eine im wesentlichen konstante, auf die Handgriffe 12, 13 der Zange wirkende Kraft in Abhängigkeit der Wegstrecke b′ dargestellt, die der Handgriff 12 gegenüber dem Handgriff 13 bei Verkleinerung des Abstandes b zwischen den Handgriffen 12, 13 zurücklegt. Mit kleiner werdendem Abstand b zwischen den Handgriffen 12, 13, also mit größer werdender Wegstrecke b′, nimmt die auf die Schneiden wirkende Kraft zu, wobei der Kraftzuwachs der Vergrößerung der Auflageflächen der Schneiden 15, 15′ am Draht 14 entspricht, so daß die Schneiden stets zum Trennen des Drahtes 14 erforderlichen Druck auf diesen ausüben.

Sollen mit der beschriebenen Zange Drähte oder Schrauben mit größerem Durchmesser durchgetrennt werden, dann wird eine Erhöhung der auf die Schneiden 15, 15′ wirkenden Kraft schon erforderlich, wenn der Schließwinkel zwischen dem Schneiden-Griffhebel 1 und dem Griffhebel 4 und damit der Abstand b zwischen den Handgriffen 12, 13 noch groß ist und die automatische Erhöhung der Kraftübersetzung praktisch noch nicht eintritt.

In diesem Fall wird beim Zusammendrücken der Handgriffe 12, 13 das vordere Ende des Übertragungshebels 6 und damit das Kniegelenk 11′ des zweiten Kniehebels 8, 10 nach vorne geschoben, ohne daß das Zwischengelenk 11 des Zwischenhebels 10 dieser Bewegung folgt, da zunächst der Federschlitz 18 des federnden Zwischenhebels 10 gegen die von diesem ausgeübte Federkraft zusammengedrückt wird. Demzufolge ändert sich der Abstand a zwischen den Schneiden 15, 15′ zunächst nicht. Sobald der Federschlitz 18 im Zwischenhebel 10 geschlossen ist, bewirkt die weitere Verschiebung des Kniegelenks 11′ in Abhängigkeit von der Verringerung des Abstandes b zwischen den Handgriffen 12, 13 eine Verschiebung des Zwischengelenks 11 und damit ein Schließen der Schneiden 15, 15′. In diesem Bereich liegt nun bereits ein vergrößertes Kraftübersetzungsverhältnis vor, so daß die automatische Kraftsteigerung voll zur Wirkung kommt und der von den Schneiden 15, 15′ auf den Draht oder die Schraube ausgeübte Druck stets zumindest gleich oder größer als der erforderliche Trenndruck ist. Gleichzeitig wird es ermöglicht, daß der Abstand zwischen den Handgriffen 12, 13 weiter verkleinert werden kann, als dies der Fall wäre, wenn ein starrer Zwischenhebel verwendet werden würde, so daß eine zusätzliche zur Druckerzeugung erforderliche Kraftvergrößerung ermöglicht wird.

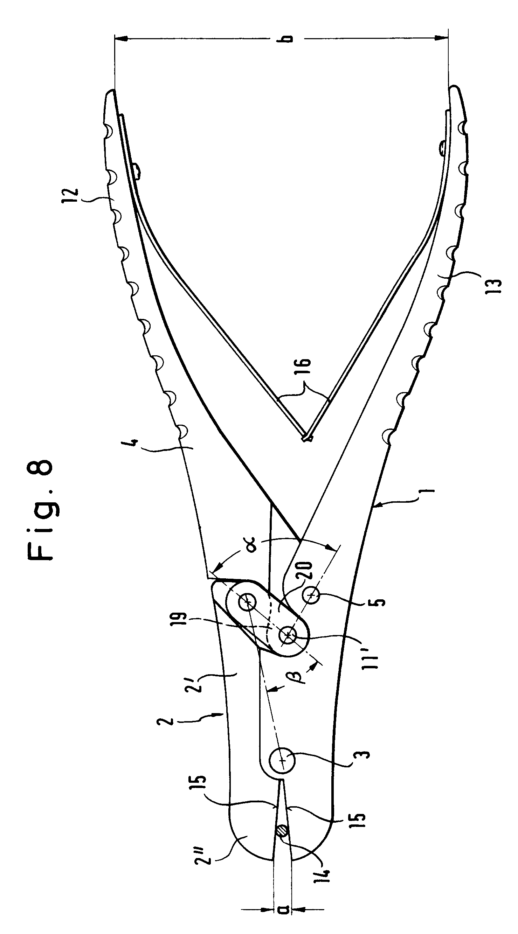

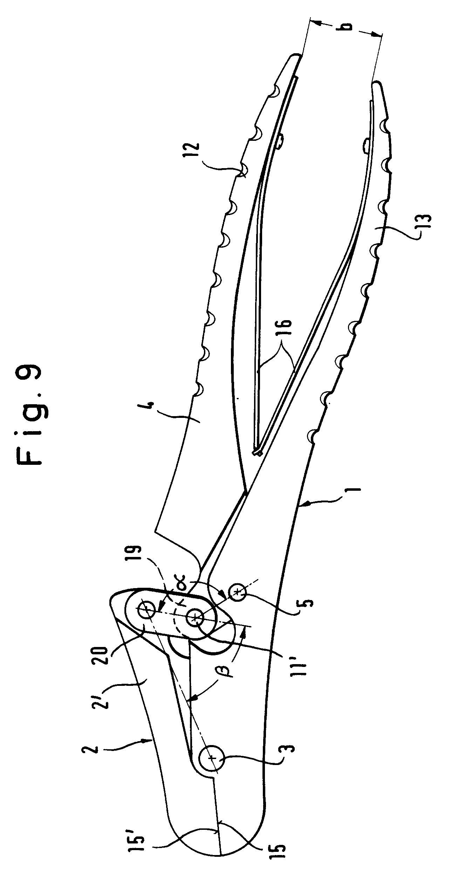

Das Ausführungsbeispiel nach den Fig. 8 und 9 unterscheidet sich von dem vorangehenden zunächst dadurch, daß der Abstand zwischen dem Schneidenhebelgelenk 3 und dem Griffhebelgelenk 5 wesentlich vergrößert ist. Außerdem ist der Griffhebel 4 jenseits des Griffhebelgelenks 5 in Richtung des Schneidenhebelgelenks 3 durch einen relativ kurzen Kniehebelarm 19 verlängert, an welchem über ein Kniegelenk 11′ eine Kniehebellasche 20 angelenkt ist, deren anderes Ende gelenkig mit dem hinteren Hebelarm 2′ des Schneidenhebels 2 gelenkig verbunden ist.

Auf diese Weise ist zwischen den Griffhebel 4 und den Schneidenhebel 2 ein Kniehebelgetriebe bestehend aus dem Kniehebel 19, 20 geschaltet, wobei der Knickwinkel des Kniehebels 19, 20 in der geöffneten Position der Zange nach Fig. 8 einen Knickwinkel α von etwas kleiner als 90° aufweist, während in der zusammengedrückten Position nach Fig. 9 der Knickwinkel α bei 135° liegt.

Aufgrund dieser Ausbildung wird am Beginn der Zuspannbewegung der Zange gemäß Fig. 8 zunächst eine vergleichsweise geringe Kraft auf den eingespannten Draht 14 ausgeübt. Mit zunehmender Streckung des Kniehebels 19, 20 nimmt die Kraftübersetzung jedoch im wesentlichen quadratisch zu, so daß gerade dann, wenn der Draht an seiner in der Seitenansicht nach Fig. 8 breitesten Stelle durchschnitten werden soll, eine ganz erheblich vergrößerte Schneidkraft zur Verfügung steht. Hierzu ist es auch wichtig, daß der Winkel β zwischen dem Hebelarm 2 und der Kniehebellasche 20, der nach Fig. 8 ca. 35° beträgt, sich bis auf annähernd 60° (Fig. 9) vergrößert.

Die Rückstellkraft für die Zange wird durch die aus Fig. 8 und 9 ersichtliche Doppelblattfeder 16 erzielt, welche aus zwei vom freien Ende der Handgriffe 12 bzw. 13 sich in Richtung des Gelenks 5 erstreckenden Federblättern besteht, deren dem Gelenk 5 zugewandte Enden gelenkig miteinander verbunden sind.

Die Achsen sämtlicher bei der erfindungsgemäßen Zange vorhandenen Gelenke, insbesondere des Schneidenhebelgelenks 3, des Griffhebelgelenks 5 sowie der verschiedenen Kniehebelgelenke stehen senkrecht auf der durch den Schneidengriffhebel 1 und den Schneidenhebel 2 definierten Ebene.

## Patentansprüche

1. Zange, insbesondere für chirurgische Zwecke, zum Durchtrennen von Drähten, Schrauben o.dgl. mit einem an seinem vorderen Ende eine Schneide (15) und an seinem hinteren Ende einen Handgriff (13) aufweisenden Schneiden-Griffhebel (1), an dem ein eine zweite, der ersten Schneide (15) gegenüberliegende Schneide (15') aufweisender Schneidenhebel (2) mittels eines Schneidenhebelgelenks (3) sowie dahinter ein Griffhebel (4) mittels eines Griffhebelgelenks (5) schwenkbar angelenkt sind, wobei zwischen dem Griffhebel (4) und dem Schneidenhebel (2) ein Getriebe angeordnet ist, dadurch
**gekennzeichnet,** daß das Antriebsglied (6) des Getriebes vom Griffhebel (4) beaufschlagt wird und daß das Abtriebsglied (10) des Getriebes auf der von der Schneide (15') abgewandten Seite eines den Schneidenhebel (2) am Schneiden-Griffhebel (1) abstützenden Schneidenhebelgelenks (3) am Schneidenhebel (2) angreift, wobei gleiche Relativverschwenkungen des Griffhebels (4) gegenüber dem Schneiden-Griffhebel (1) eine deutlich geringer werdende Verschwenkung des Schneidenhebels (2) gegenüber dem Schneiden-Griffhebel (1) bewirken, so daß die Kraftübersetzung automatisch mit kleiner werdendem Abstand (b) zwischen den jeweils am Griffhebel (4) und am Schneiden-Griffhebel (1) ausgebildeten Handgriffen (12, 13) zunimmt.

2. Zange nach Anspruch 1 dadurch
**gekennzeichnet,** daß als Getriebe ein Kniehebelantrieb (6, 8, 10) vorgesehen ist, dessen Antriebsglied von einem mit seinem ersten Ende am Griffhebel (4) angelenkten Übertragungshebel (6) gebildet ist, der zusammen mit dem Griffhebel (4) einen ersten Kniehebel (4, 6) bildet und der mit seinem anderen Ende ein Kniegelenk (11') eines zweiten Kniehebels (8, 10) beaufschlagt, welcher aus einem am Schneiden-Griffhebel (1) angelenkten Stützhebel (8) und einem das Abtriebsglied (10) darstellenden Zwischenhebel besteht, der sich vom Kniegelenk (11') zum Schneidenhebel (2) erstreckt, an dem er in einem Zwischengelenk (11) angelenkt ist.

3. Zange nach Anspruch 2, dadurch
**gekennzeichnet,** daß zwischen dem Griffhebel (4) und dem Übertragungshebel (6) ein bei Betätigung der Zange sich verkleinernder, spitzer Winkel und zwischen dem Stützhebel (8) und dem Zwischenhebel (10) ein bei Betätigung der Zange sich vergrößernder, stumpfer Winkel vorgesehen ist, wobei bei geschlossenen Schneiden (15, 15') der Übertragungswinkel (6) nahezu parallel zum Griffhebel (4) und der zweite Kniehebel (8, 10) kurz vor seiner Strecklage liegt.

4. Zange nach Anspruch 2 oder 3, dadurch
**gekennzeichnet,** daß die Länge des Zwischenhebels (10) verkleinerbar ist.

5. Zange nach Anspruch 4, dadurch
**gekennzeichnet,** daß der Zwischenhebel (10) als Feder ausgebildet ist.

6. Zange nach Anspruch 5, dadurch
**gekennzeichnet,** daß der Zwischenhebel (10) von einer U-förmigen Druckfeder gebildet ist, deren Federschlitz (18) die Längenveränderung des Zwischenhebels (10) begrenzt.

7. Zange nach Anspruch 1, dadurch
**gekennzeichnet,** daß der Griffhebel (4) jenseits des Griffhebelgelenks (5) durch einen relativ kurzen Kniehebelarm (19) verlängert ist, der über ein Kniegelenk (11') eine zusammen mit dem Kniehebelarm (19) einen Kniehebel (19, 20) bildenden Kniehebellasche (20) beaufschlagt, die mit dem vom Kniegelenk (11') abgewandten Ende an dem bezüglich des Schneidenhebelgelenks (3) von der Schneide (15) abgewandten Hebelarm (2') des Schneidenhebels (2) gelenkig verbunden ist, wobei der Kniehebel (19, 20) beim Verringern des Abstandes (b) zwischen den Handgriffen (12, 13) von einer stark geknickten in eine mehr gestreckte Position übergeht und die Kniehebellasche (20) im geöffneten Zustand der Zange einen deutlichen Winkel von vorzugsweise wenigstens 30 bis 40°, insbesondere 35°, mit dem besagten Hebelarm (2') einschließt, welcher sich zweckmäßig bei aufeinander zu bewegenden Schneiden (15, 15') immer mehr an 90° annähert und/oder bei geschlossenen oder annähernd geschlossenen Schneiden (15, 15') bevorzugt 40-80°, zweckmäßig 60-70° und insbesondere etwa 60° beträgt.

8. Zange nach Anspruch 7, dadurch
**gekennzeichnet,** daß der Kniehebel (19, 20) im unbetätigten Zustand der Zange einen Knickwinkel (α) von größenordnungsmäßig 90° und vorzugsweise etwas weniger als 90°, insbesondere etwa 80 bis 90° aufweist und sich im vollständig zusammengedrückten Zustand der Zange nahe seinem gestreckten Zustand befindet und bevorzugt einen Knickwinkel (α) zwischen 120° und 150°, bevorzugt 130° bis 140° aufweist.

9. Zange nach Anspruch 7 oder 8, dadurch
**gekennzeichnet,** daß der Hebelarm (2') zwischen dem Schneidenhebelgelenk (3) und der Kniehebellasche (20) um einen Faktor 1,5 bis 2 länger ist als der die Schneide (15') tragende Hebelarm (2'').

## Claims

1. Pliers, in particular for surgical purposes for the cutting through of wires, screws or the like, comprising a cutter gripping lever (1) having a cutting edge (15) at its front end and a handle (13) at its rear end, the cutter gripping lever (1) being pivotably connected by means of a cutting lever hinge (3) to a cutting lever (2) which has a second cutting edge (15') disposed opposite to the first cutting edge (15), as well as being pivotably connected by means of a gripping lever hinge (5) to a gripping lever (4) behind the cutting lever hinge (3) and wherein a transmission is arranged between the gripping lever (4) and the cutting lever (2), characterised in that the drive member (6) of the transmission is actuated by the gripping lever (4); and in that the driven member (10) of the transmission acts on the cutting lever (2) on the side of the cutting lever hinge (3) which is remote from the cutting edge (15'), with the cutting lever hinge (3) supporting the cutting lever (2) at the cutter gripper lever (1) and wherein like relative pivotal movements of the gripping lever (4) relative to the cutter gripping lever (1) bring about progressively smaller pivotal movements of the cutting lever (2) relative to the cutter gripping lever (1), so that the mechanical advantage increases automatically as the distance (b) between the handles (12, 13) formed at the gripping lever (4) and at the cuttergripping lever (1) respectively becomes smaller.

2. Pliers in accordance with claim 1, characterised in that a toggle lever drive (6, 8, 10) is provided as the transmission, the drive member of which being formed by a transmission lever (6) which is pivotally connected at its first end to the gripping lever (4), with the transmission lever together with the gripping lever (4) forming a first toggle lever (4, 6) and with the other end of the transmission lever actuating a toggle lever hinge (11') of a second toggle lever (8, 10), the second toggle lever (8,10) comprising a support lever (8) which is pivotally connected to the cutter gripping lever (1) and an intermediate lever which represents the driven member (10), with the driven member (10) extending from the toggle lever joint (11') to the cutting lever (2) at which it is pivotally connected by an intermediate hinge (11).

3. Pliers in accordance with claim 2, characterised in that an acute angle which becomes smaller on actuating the pliers is provided between the gripping lever (4) and the transmission lever (6), and in that an obtuse angle which becomes larger on actuating the pliers is provided between the support lever (8) and the intermediate lever (10), wherein, when the cutting edges (15, 15') are closed, the transmission lever (6) lies approximately parallel to the gripping lever (4) and the second toggle lever (8, 10) lies shortly before its extended position.

4. Pliers in accordance with claim 2 or 3, characterised in that the length of the intermediate lever (10) can be reduced.

5. Pliers in accordance with claim 4, characterised in that the intermediate lever (10) is formed as a spring.

6. Pliers in accordance with claim 5, characterised in that the intermediate lever (10) is formed by a U-shaped compression spring, the spring slot (18) of which limiting the change in length of the intermediate lever (10).

7. Pliers in accordance with claim 1, characterised in that the gripping lever (4) is extended on the far side of the gripping lever hinge (5) by a relatively short toggle lever arm (19) which activates a toggle lever link (20) via a toggle lever hinge (11'), with the toggle lever link (20) together with the toggle lever arm (19) forming a toggle lever (19, 20) which is pivotally connected at the end remote from the toggle lever joint (11') to the lever arm (2') of the cutting lever (2) remote from the cutting edge (15) with respect to the cutting lever hinge (3), wherein the toggle lever (19, 20) on reduction of the spacing (b) between the handles (12, 13) passes from a greatly kinked position into a more extended position and wherein, in the opened state of the pliers, the toggle lever link (20) forms a substantial angle of preferably at least 30 to 40°, in particular 35° with the said lever arm (2'), with this angle expediently progressively approaching 90° as the cutting edges (15, 15') move together and/or preferably amounting to 40 to 80°, expediently 60 to 70° and in particular approximately 60°, when the cutting edges (15, 15') are closed or are approximately closed.

8. Pliers in accordance with claim 7, characterised in that in the non-actuated state of the pliers the toggle lever (19, 20) has a kink angle (α) of the order of magnitude of 90° and preferably somewhat less than 90°, in particular approximately 80 to 90° and in that in the fully pressed-together state of the pliers the toggle lever (19, 20) is close to its extended state and preferably has a kink angle (α) of between 120° and 150°, preferably 130 to 140°.

9. Pliers in accordance with claim 7 or 8, characterised in that the lever arm (2') between the cutting lever hinge (3) and the toggle lever link (20) is longer by a factor 1.5 to 2 than the lever arm (2'') carrying the cutting edge (15').

## Revendications

1. Pince, notamment à usage chirurgical, pour sectionner des fils métalliques, vis ou éléments analogues, avec un tranchant (15) à son extrémité avant et une branche de préhension à tranchants (1) présentant une poignée (13) à son extrémité arrière, branche à laquelle sont articulés en pivotement un levier à tranchants (2), présentant un second tranchant (15') opposé au premier tranchant (15), au moyen d'une articulation de levier à tranchants (3), ainsi que ci-derrière une branche de préhension (4) au moyen d'une articulation de branche de préhension (5), un moyen de transmission étant disposé entre la branche de préhension (4) et le levier à tranchants (2), pince caractérisée en ce que l'élément de commande (6) du moyen de transmission est sollicité par la branche de préhension (4) et en ce que l'élément commandé (10) du moyen de transmission agit sur le levier à tranchants (2) du côté de l'articulation de levier à tranchants (3) du levier à tranchants (2) supportée par la branche de préhension à tranchants (1) opposé au tranchant (15'), des balayages horizontaux relatifs semblables de la branche de préhension (4) vis-à-vis de la branche de préhension à tranchants (1) occasionnant un balayage horizontal du levier à tranchants (2) vis-à-vis de la branche de préhension à tranchants (1) devenant sensiblement plus petit, de sorte que la transmission de force augmente automatiquement avec un écart (b) entre les poignées (12,13) réalisées respectivement sur la branche de préhension (4) et sur la branche de préhension à tranchants (1) devenant plus faible.

2. Pince selon la revendication 1, caractérisée en ce qu' il est prévu une commande à levier à genouillère (6,8,10) en tant que moyen de transmission, dont l'élément de commande est formé d'un levier de transmission (6) articulé par sa première extrémité à la branche de préhension (4), levier de transmission (6) qui forme ensemble avec la branche de transmission (4) un premier levier à genouillère (4,6) et qui sollicite avec son autre extrémité une articulation à genouillère (11') d'un second levier à genouillère (8,10), second levier à genouillère qui est constitué d'un levier d'appui (8) articulé à la branche de préhension à tranchants (1) et d'un levier intermédiaire constituant l'élément commandé (10), levier intermédiaire qui s'étend de l'articulation à genouillère (11') vers le levier à tranchants (2) auquel il est articulé dans une articulation intermédiaire (11).

3. Pince selon la revendication 2, caractérisée en ce que, entre la branche de préhension (4) et le levier de transmission (6) est prévu un angle aigu rapetissant lors de l'actionnement de la pince et entre le levier d'appui (8) et le levier intermédiaire (10) est prévu un angle obtus s'agrandissant lors de l'actionnement de la pince le levier de transmission (6) étant placé à peu près parallèle à la branche de préhension (4) et le second levier à genouillère (8,10) étant placé juste devant sa position d'extension à l'état serré des tranchants (15,15').

4. Pince selon la revendication 2 ou 3, caractérisée en ce que la longueur du levier intermédiaire (10) est réductible.

5. Pince selon la revendication 4, caractérisée en ce que le levier intermédiaire (10) se présente sous la forme d'un ressort.

6. Pince selon la revendication 5, caractérisée en ce que le levier intermédiaire (10) est formé d'un ressort de pression en forme de U, dont la fente de ressort (18) délimite le changement de longueurs du levier intermédiaire (10).

7. Pince selon la revendication 1, caractérisée en ce que la branche de préhension (4) est prolongée au-delà de l'articulation de branche de préhension (3) par un bras de levier à genouillère (19) relativement court qui sollicite une plaquette de levier à genouillère (20) formant ensemble avec le bras de levier à genouillère (19) un levier à genouillère (19,20) par l'intermédiaire d'une articulation à genouillère (11'), plaquette qui est reliée, articulée par son extrémité opposée à l'articulation à genouillère (11') au bras de levier (2') du levier à tranchants (2) opposé au tranchant (15) par rapport à l'articulation de levier à tranchants (3), le levier à genouillère (19,20) passant, lors d'une diminution de l'écart (b) entre les poignées (12,13), d'une position fortement pliée à une position plus étendue et la plaquette de levier à genouillère (20) définissant avec ledit bras de levier (2') un angle marqué davantageusement d'au moins 30 à 40°, plus particulièrement de 35°, angle qui de façon appropriée, lors d'un déplacement des tranchants (15,15') pour venir porter l'un sur l'autre, se rapproche de plus en plus de 90° et/ou à l'état serré ou approximativement serré des tranchants (15,15') fait préférentiellement de 40 à 80°, de façon appropriée 60 à 70° et plus particulièrement environ 60°

8. Pince selon la revendication 7, caractérisée en ce que le levier à genouillère (19,20) présente, à l'état non actionné de la pinae, un angle de brisure (α) dont l'ordre de grandeur est 90° et avantageusement un peu moins de 90°, plus particulièrement environ 80 à 90°, et qui à l'état entièrement serré de la pince se trouve proche de son état étendue et présente avantageusement un angle de brisure (α) entre 120° et 150°, préférentiellement entre 130° et 140°

9. Pince selon la revendication 7 ou 8, caractérisée en ce que le bras de levier (2') entre l'articulation de levier à tranchants (3) et la plaquette de levier à genouillère (20) est plus long d'un facteur 1,5 à 2 par rapport au bras de levier (2'') portant le tranchant (15').
